# EUROPÄISCHE PATENTSCHRIFT

(11) **EP 2 823 793 B1**
(45) Veröffentlichungstag und Bekanntmachung des Hinweises auf die Patenterteilung: **19.04.2017**
(21) Anmeldenummer: 14175709.6
(22) Anmeldetag: 04.07.2014
(51) Int. Cl.: A61F 2/915

(54) **Stent für gewundene Blutgefässe**
Stent for tortuous blood vessels
Endoprothèse pour vaisseaux contournés

(30) Priorität: 09.07.2013 DE 102013107258
(43) Veröffentlichungstag der Anmeldung: 14.01.2015
(73) Patentinhaber: Acandis GmbH & Co. KG, 76327 Pfinztal (DE)
(72) Erfinder: Nagl, Frank, 76137 Karlsruhe (DE); Klopp, David, 75196 Remchingen (DE)
(74) Vertreter: Meissner Bolte Partnerschaft mbB

(56) Entgegenhaltungen:
- WO-A1-2013/134560
- WO-A2-03/075797
- DE-B3-102011 009 371
- US-A1- 2009 171 426

## Beschreibung

Die Erfindung betrifft einen Stent gemäß dem Oberbegriff des Patentanspruchs 1. Ein derartiger Stent ist aus DE 10 2011 009 371 B3 bekannt. Der Stent weist eine komprimierbare und selbstexpandierbare, kreiszylinderförmige Gitterstruktur mit geschlossenen Zellen aus vier Stegen auf. Die Stege sind an Verbindungsstellen miteinander gekoppelt und unterschiedlich flexibel ausgebildet. Ähnliche medizinische Vorrichtungen sind aus der Praxis bekannt und beispielsweise in US 2009/171426 A1 oder WO 03/075797 A2 beschrieben.

Stents werden in unterschiedlichen Körpergefäßen eingesetzt und nehmen verschiedene Aufgaben wahr. Einerseits werden Stents genutzt, um verengte Gefäße aufzuweiten und so den vollständigen Blutfluss wiederherzustellen. Andererseits dienen Stents auch dazu, die Blutströmung zu beeinflussen, beispielsweise um den Blutstrom in ein Aneurysma zu unterbinden oder zumindest zu reduzieren. Ein generelles Prinzip für den Einsatz von Stents besteht darin, dass Stents in einem komprimierten Zustand über einen Katheter an den Behandlungsort geführt werden. Am Behandlungsort werden die Stents dann expandiert, um sich an die Kontur des jeweiligen Gefäßes anzupassen.

Schwierigkeiten ergeben sich bei der Implantation von Stents in stark gewundenen Gefäßabschnitten. Um die Stents in derartige Gefäßabschnitte zu bringen, ist man bestrebt, das Stent-Design derart zu gestalten, dass der Stent möglichst biegeflexibel ist. Dies kann dazu führen, dass sich der Stent bei der Expansion unerwartet verhält und insbesondere wegen der hohen Flexibilität dazu tendiert zu tordieren. Gleichzeitig soll sich die hohe Flexibilität des Stents nicht auf die längsaxiale Steifigkeit auswirken, die benötigt wird, um den Stent durch einen Katheter an den Behandlungsort zu führen.

Die Aufgabe der Erfindung besteht darin, eine medizinische Vorrichtung zur Einfuhr in ein Körperhohlorgan, insbesondere einen Stent, anzugeben, der sich gut an gewundene Gefäßabschnitte anpasst und gleichzeitig ein gutes Zuführverhalten und Expansionsverhalten zeigt.

Erfindungsgemäß wird diese Aufgabe durch den Gegenstand des Patentanspruchs 1 gelöst.

So beruht die Erfindung auf dem Gedanken einen Stent mit einer komprimierbaren und selbstexpandierbaren, rohrförmigen Gitterstruktur aus Stegen anzugeben, wobei die Stege durch erste und zweite Stegverbinder einstückig miteinander verbunden sind und geschlossene Zellen der Gitterstruktur begrenzen. Jeweils vier Stege begrenzen eine geschlossene Zelle. Die Stegverbinder weisen jeweils eine Verbinderachse auf, die dem kürzesten Abstand zwischen zwei Zellenspitzen von in Längsrichtung am Stegverbinder gegenüberliegenden Zellen entspricht. Dabei sind in einem Ruhezustand der Gitterstruktur die Verbinderachsen der ersten Stegverbinder parallel und die Verbinderachsen der zweiten Stegverbinder in einem Winkel zu einer Längsachse der Gitterstruktur angeordnet.

Bei der Komprimierung bzw. Expansion einer Gitterstruktur ändern sich die Höhe und die Breite der einzelnen Zellen der Gitterstruktur. Das Verhältnis zwischen Zellenhöhe und Zellenbreite kann durch die Konstruktion der Stegverbinder beeinflusst werden. So hat sich gezeigt, dass Zellen, die von Stegverbindern umgeben sind, welche im Ruhezustand eine winklig zur Längsachse der Gitterstruktur angestellte Verbinderachse aufweisen, ein sich während der Expansion der Gitterstruktur dynamisch änderndes Verhältnis zwischen Zellenhöhe und Zellenbreite aufweisen. Dies führt insbesondere dazu, dass derartige Zellen eine relativ hohe Flexibilität aufweisen. Mit anderen Worten erhöht der zweite Stegverbinder der Gitterstruktur die Flexibilität, insbesondere die Biegeflexibilität, des erfindungsgemäßen Stents.

Stegverbinder, deren Verbinderachse im Ruhezustand parallel zur Längsachse der Gitterstruktur angeordnet ist, gewährleisten hingegen eine längsaxiale Steifigkeit der Gitterstruktur. Dies ist deswegen möglich, da sich die in Längsrichtung benachbart angeordneten Zellen und Stege im Bereich des Stegverbinders mit der parallel zur Längsachse ausgerichteten Verbinderachse gegenseitig abstützen.

Indem erfindungsgemäß erste und zweite Stegverbinder, also flexible und längsaxial steife Stegverbinder, in einer gemeinsamen Gitterstruktur kombiniert sind, weist der erfindungsgemäße Stent einerseits die Vorteile einer flexiblen Gitterstruktur auf, die sich gut in gewundene Gefäße führen und implantieren lässt. Andererseits bewirken die ersten Stegverbinder mit der parallel zur Längsachse ausgerichteten Verbinderachse eine gute längsaxiale Steifigkeit und somit ein gleichmäßiges Expansionsverhalten.

Die vorgenannten Vorteile kommen besonders gut zum Tragen, wenn, wie es in einer besonders bevorzugten Ausführungsform der Erfindung vorgesehen ist, jeweils in Umfangsrichtung der Gitterstruktur benachbarte erste Stegverbinder einen ersten Verbinderring und jeweils in Umfangsrichtung der Gitterstruktur benachbarte zweite Stegverbinder einen zweiten Verbinderring bilden. So ist sichergestellt, dass in Umfangsrichtung benachbarte Stegverbinder jeweils die identische Funktion haben, wobei die ersten Stegverbinder eine Stabilisierungsfunktion und die zweiten Stegverbinder eine Flexibilitätsfunktion wahrnehmen.

Die ersten Stegverbinder, insbesondere der erste Verbinderring, können an wenigstens einem axialen Ende der Gitterstruktur angeordnet sein. Vorzugsweise ist an den axialen Enden der Gitterstruktur jeweils ein, insbesondere ein einziger, erster Verbinderring angeordnet. Auf diese Weise wird eine besonders gute Stabilisierung der Gitterstruktur erreicht, so dass ein gleichmäßiges Expansionsverhalten sichergestellt ist. Insbesondere bewirken die an den axialen Enden der Gitterstruktur angeordneten ersten Verbinderringe, also Verbinderringe mit einer Stabilisierungsfunktion, dass Torsionseffekte, die sich durch die zweiten Stegverbinder in der Gitterstruktur einstellen können, kompensiert werden. Insbesondere wird vermieden, dass die gesamte Gitterstruktur bei der Expansion in sich verdreht wird. So ist unter anderem auch gewährleistet, dass an den axialen Enden der Gitterstruktur ggf. vorgesehene Röntgenmarkierungselemente ihre Lage bei der Expansion der Gitterstruktur beibehalten.

Im Hinblick auf die Verbinderachse ist vorgesehen, dass diese den kürzesten Abstand zwischen zwei Zellenspitzen von in Längsrichtung am Stegverbinder gegenüberliegenden Zellen entspricht. Konkret können die Zellenspitzen in Längsrichtung benachbarter Zellen, die sich einen Stegverbinder teilen, im Ruhezustand der Gitterstruktur fluchtend zueinander angeordnet sein. Dies entspricht einem ersten Stegverbinder mit Stabilisierungsfunktion. Die zweiten Stegverbinder verbinden hingegen in Längsrichtung benachbarte Zellen, deren am Stegverbinder angeordnete Zellenspitzen in Umfangsrichtung versetzt zueinander angeordnet sind. Dies gilt für den Ruhezustand der Gitterstruktur. In beiden Fällen ist die Verbinderachse als kürzester Abstand zwischen zwei an einem Stegverbinder benachbarten Zellenspitzen definiert, wobei die Zellenspitzen von in Längsrichtung benachbarten Zellen betrachtet werden.

Konkret können in Längsrichtung benachbarte Zellen der Gitterstruktur jeweils eine Zellenspitze mit einer Krümmung umfassen, wobei die Krümmung einen Scheitelpunkt aufweist, der einen Endpunkt der Verbinderachse bildet. Stege der Gitterstruktur, die in Umfangsrichtung der Gitterstruktur benachbart zueinander angeordnet sind, sind an ihren Längsenden vorzugsweise in einem Stegverbinder zusammengeführt. Dabei kann sich zwischen den in Umfangsrichtung benachbarten Stegen eine Krümmung bilden, die gleichzeitig eine Teilbegrenzung der zugehörigen Zelle darstellt. Insbesondere begrenzt die Krümmung eine Zellenspitze, vorzugsweise die Zellenspitze einer geschlossenen Zelle.

Die Zelle kann eine rautenförmige Grundform aufweisen. Die Ecken der rautenförmigen Grundform bilden jeweils die Zellenspitzen. Im Allgemeinen müssen die Zellenspitzen im Rahmen der vorliegenden Anmeldung nicht spitz im Sinne einer scharfen Begrenzung sein, sondern können eine Krümmung aufweisen. Die Krümmung kann insbesondere parabelförmig ausgebildet sein, so dass sich ein Scheitelpunkt ergibt, der sich im Maximum der Parabel befindet. Dieser Scheitelpunkt ist gleichzeitig der Endpunkt der Verbinderachse.

Bei einer weiteren bevorzugten Ausführungsform der Erfindung ist vorgesehen, dass die zweiten Stegverbinder beim Übergang der Gitterstruktur vom Ruhezustand in einen komprimierten Zustand derart rotieren, dass sich ein Winkel zwischen der Verbinderachse und der Längsachse der Gitterstruktur ändert, insbesondere zumindest teilweise erhöht. Dies trägt zur Erhöhung der Flexibilität der Gitterstruktur bei. Insbesondere ermöglichen die in den Stegverbindern individuell möglichen Rotationsgrade, dass sich die Gitterstruktur im Bereich der zweiten Stegverbinder ovalisieren kann. Die Gitterstruktur kann also bereichsweise einen ovalen Querschnitt einnehmen. So kann die Gitterstruktur leicht durch gewundene Gefäßabschnitte geführt oder in gewundene Gefäßabschnitte implantiert werden.

In einer Ausgestaltung der Erfindung sind in einem mittleren Bereich der Gitterstruktur, insbesondere zwischen den axialen Enden bzw. zwischen den ersten Verbinderringen, jeweils ein oder mehrere zweite Stegverbinder, insbesondere ein oder mehrere zweite Verbinderringe, angeordnet.

Zwischen einem ersten Verbinderring und einem zweiten Verbinderring kann außerdem ein Übergangsverbinderring angeordnet sein. Dabei bilden die Stegverbinder des Übergangsverbinderrings in einem Ruhezustand der Gitterstruktur einen kleineren Winkel mit der Längsachse der Gitterstruktur als die Stegverbinder des zweiten Verbinderrings. Die Stegverbinder im Übergangsverbinderring können ebenfalls eine Rotationsfähigkeit aufweisen, die jedoch geringer als bei den Stegverbindern des zweiten Verbinderrings ausgeprägt ist. Der Übergangsverbinderring ermöglicht einen sanften Übergang von längsaxial stabilisierenden ersten Stegverbindern und flexibilitätserhaltenden zweiten Stegverbindern.

Ferner können die axialen Enden der Gitterstruktur Endzellen aufweisen, die jeweils zwei verlängerte Stege umfassen. Die zwei verlängerten Stege können durch ein Markerelement miteinander verbunden sein. Eine derartige Konstruktion verbessert die Röntgensichtbarkeit der Gitterstruktur.

Vorzugsweise sind axiale Endabschnitte der Gitterstruktur konusförmig aufgeweitet. Dies trägt zusätzlich zur verbesserten Implantation der erfindungsgemäßen medizinischen Vorrichtung in stark gewundenen Gefäßabschnitten bei. Wenn ein Endabschnitt der Gitterstruktur bei der Implantation in einer Gefäßkrümmung zu liegen kommt, ermöglicht der konusförmig aufgeweitete axiale Endabschnitt, dass sich die Stege im Endabschnitt der Gitterstruktur gut an die Gefäßwand anlegen. Insbesondere wird vermieden, dass die Stege in den Strömungskanal hineinragen.

Im Hinblick auf die Dimensionen der Endabschnitte ist bevorzugt vorgesehen, dass diese jeweils eine Länge aufweisen, die höchstens dem 0,3-fachen, insbesondere dem 0,25-fachen, insbesondere höchstens dem 0,15-fachen, der Gesamtlänge der Gitterstruktur entspricht.

Um die Flexibilität der Gitterstruktur weiter zu verbessern, kann vorgesehen sein, dass die Stege der einzelnen Zellen unterschiedliche Stegbreiten aufweisen. Beispielsweise können jeweils diametral gegenüberliegende bzw. parallel verlaufende Stege einer rautenförmigen Zelle ein Stegpaar bilden, wobei ein erstes Stegpaar der Zelle eine erste Stegbreite und ein zweites Stegpaar derselben Zelle eine zweite Stegbreite aufweist. Dies unterstützt die Rotationsfähigkeit der zweiten Stegverbinder und trägt somit zur Verbesserung der Gesamtflexibilität der Gitterstruktur bei.

Die Gitterstruktur ist selbstexpandierbar ausgebildet. Konkret kann die Gitterstruktur ein superelastisches Material, insbesondere eine Nickel-Titan-Legierung, vorzugsweise Nitinol, aufweisen oder daraus bestehen. Auf diese Weise kann die Flexibilität der erfindungsgemäßen medizinischen Vorrichtung durch die Verwendung eines geeigneten Materials, vorzugsweise eine Nickel-Titan-Legierung, weiter erhöht werden.

Die Erfindung wird im Folgenden anhand eines Ausführungsbeispiels unter Bezugnahme auf die beigefügten, schematischen Zeichnungen näher erläutert.

Darin zeigen
- Fig. 1: eine Abrollansicht einer erfindungsgemäßen medizinischen Vorrichtung nach einem bevorzugten Ausführungsbeispiel;
- Fig. 2: eine Detailansicht der Vorrichtung gemäß Fig. 1;
- Fig. 3: eine Draufsicht eines ersten Stegverbinders der Vorrichtung gemäß Fig. 1;
- Fig. 4: eine Seitenansicht der Vorrichtung gemäß Fig. 1;
- Fig. 5: eine Seitenkonturansicht der Vorrichtung gemäß Fig. 1;
- Fig. 6: eine Draufsicht auf eine Zelle mit ersten Stegverbindern der Vorrichtung gemäß Fig. 1;
- Fig. 7: eine Seitenansicht der Vorrichtung gemäß Fig. 1 im implantierten Zustand mit einem zylinderförmigen Endabschnitt; und
- Fig. 8: eine Seitenansicht der Vorrichtung gemäß Fig. 1 im implantierten Zustand mit einem konusförmigen Endabschnitt.

Die beigefügten Figuren zeigen einen Stent, der zur Einfuhr in ein Körperhohlorgan geeignet ist. Die medizinische Vorrichtung weist dazu insbesondere eine Gitterstruktur 10 auf, die komprimierbar ist. Mit anderen Worten kann die Gitterstruktur 10 einen Zuführzustand einnehmen, in dem die Gitterstruktur 10 einen relativ kleinen Querschnittsdurchmesser aufweist. Die Gitterstruktur 10 ist selbstexpandierbar ausgebildet, so dass die Gitterstruktur 10 sich ohne Einfluss äußerer Kräfte selbsttätig auf einen maximalen Querschnittsdurchmesser aufweitet. Der Zustand, in dem die Gitterstruktur 10 den maximalen Querschnittsdurchmesser aufweist, entspricht dem Ruhezustand. In diesem Zustand übt die Gitterstruktur 10 keinerlei Radialkräfte aus.

Vorzugsweise ist die Gitterstruktur 10 einstückig ausgebildet. Insbesondere kann die Gitterstruktur 10 zumindest abschnittsweise zylinderförmig ausgebildet sein. Die Gitterstruktur 10 ist vorzugsweise aus einem rohrförmigen Rohling durch Laserschneiden hergestellt. Dabei werden einzelne Stege 11, 12, 13, 14 der Gitterstruktur durch die laserschneidende Bearbeitung freigelegt. Die aus dem Rohling entfernten Bereiche bilden Zellen 16 der Gitterstruktur 10.

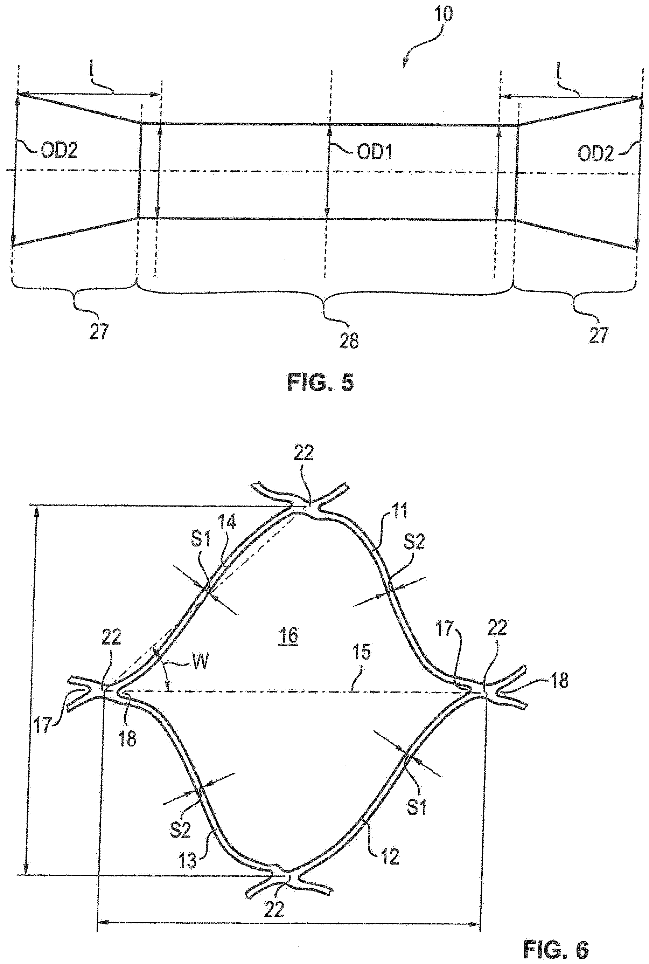

Die Zellen 16 weisen im Wesentlichen eine rautenförmige Grundform auf. Dies ist in Fig. 6 gut erkennbar. Insbesondere sind die Zellen 16 durch jeweils vier Stege 11, 12, 13, 14 begrenzt, wobei die Stege 11, 12, 13, 14 zumindest teilweise einen gekrümmten, insbesondere S-förmigen, Verlauf aufweisen können. Die Zellen 16 weisen jeweils Zellenspitzen 17, 18 auf, die die Eckpunkte der rautenförmigen Grundform festlegen. Die Zellenspitzen 17, 18 sind jeweils an Stegverbindern 21, 22, 25 angeordnet, die jeweils vier Stege 11, 12, 13, 14 einstückig miteinander verbinden. Von jedem Stegverbinder 21, 22, 25 gehen jeweils vier Stege 11, 12, 13, 14 aus, wobei jeder Steg 11, 12, 13, 14 jeweils zwei Zellen 16 zugeordnet ist. Die Stege 11, 12, 13, 14 begrenzen jeweils die Zellen 16.

Die Stegverbinder 21, 22, 25 verbinden jeweils vier Stege 11, 12, 13, 14 miteinander. Der Einfachheit halber werden im Rahmen der Anmeldung die an einem Stegverbinder 21, 22, 25 zusammengeführten Stege 11, 12, 13, 14 im Gegenuhrzeigersinn durchnummeriert, um eine klare Zuordnung zu schaffen. Dabei ist vorgesehen, dass ein erster Steg 11 und ein zweiter Steg 12 einer ersten Zelle 16 und ein dritter Steg 13 und ein vierter Steg 14 einer zweiten Zelle 16 zugeordnet sind, wobei die Zellen 16 in Längsrichtung der Gitterstruktur 10 unmittelbar benachbart sind. Mit anderen Worten trennt der Stegverbinder 21, 22, 25 zwei in Längs- und/oder Umfangsrichtung benachbart zueinander angeordnete Zellen 30 der Gitterstruktur 10 voneinander. Im Bereich des Stegverbinders 21, 22, 25 bilden die Zellen 16 jeweils die Zellenspitzen 17, 18. Die erste Zellenspitze 17 ist durch ersten Steg 11 und den zweiten Steg 12 begrenzt. Die zweite Zellenspitze 18 ist durch den dritten Steg 13 und den vierten Steg 14 begrenzt.

Die Zellenspitzen 17, 18 bilden bzw. umfassen jeweils eine Krümmung 19. Die Krümmung 19 ergibt sich durch den Übergang der Seitenflächen von in Umfangsrichtung benachbarten Stegen 11, 12, 13, 14. Konkret bildet die erste Zellenspitze 17 eine Krümmung 19, die sich aus dem Übergang des ersten Stegs 11 zum zweiten Steg 12 im Bereich des Stegverbinders 21, 22, 25 ergibt. Entsprechend ist die Krümmung 19 der zweiten Zellenspitze 18 durch einen gekrümmten Übergang zwischen dem dritten Steg 13 und dem vierten Steg 14 am Stegverbinder 21, 22, 25 geformt. Die Krümmungen 19 weisen vorzugsweise eine parabelartige Form auf. Jede der Krümmungen 19 weist ein Maximum bzw. einen Scheitelpunkt 26 auf, also einen Punkt, an welchem der kleinste Krümmungsradius vorliegt. Die Entfernung zwischen den Scheitelpunkten 26 der an einem Stegverbinder 21, 22, 25 gegenüberliegenden Krümmungen 19 entspricht dem kürzesten Abstand zwischen den Zellenspitzen 17, 18. Die Verbindungslinie, d.h. der Abstand, zwischen den beiden Maxima bzw. Scheitelpunkten 26 der Krümmungen 19 bildet die Verbinderachse 20. Mit anderen Worten entspricht die Verbinderachse 20 dem kürzesten Abstand zwischen den beiden Krümmungen 19 der ersten Zellenspitze 17 und der zweiten Zellenspitze 18.

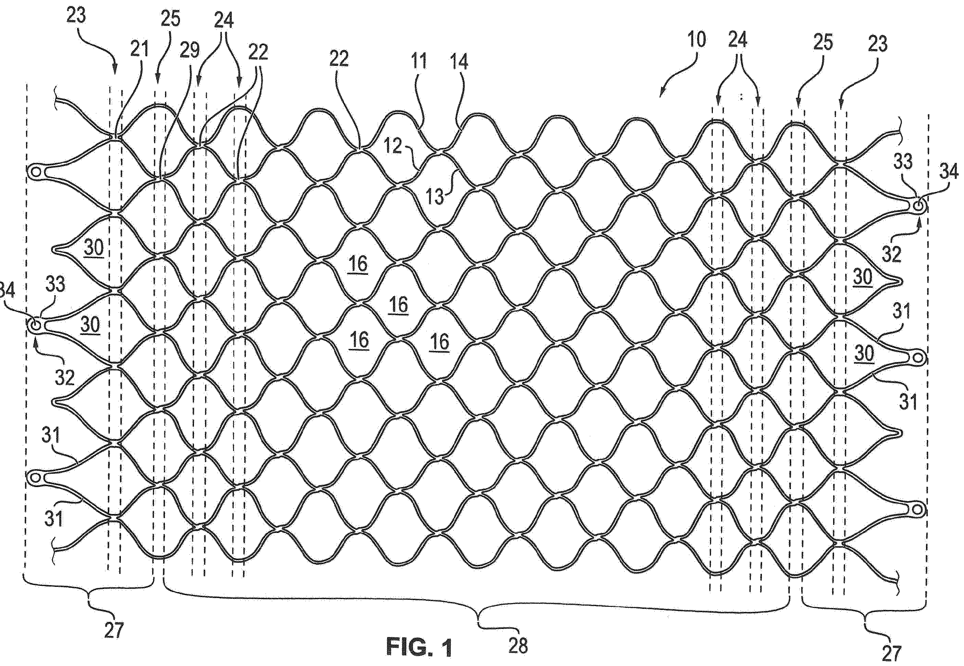

Fig. 1 zeigt die (abgerollte) Gitterstruktur 10 im Ruhezustand. Es ist gut erkennbar, dass die Stegverbinder 21, 22, 25 im Wesentlichen jeweils auf einer gemeinsamen Umfangslinie angeordnet sind. Insgesamt bilden mehrere Zellen 16 in Umfangrichtung der Gitterstruktur 10 einen Zellenring. Mehrere in Längsrichtung miteinander verbundene Zellenringe bilden die gesamte Gitterstruktur 10.

An den axialen Enden der Gitterstruktur 10 ist jeweils ein Endzellenring angeordnet. Der Endzellenring ist durch eine Vielzahl von Endzellen 30 gebildet, wobei zumindest teilweise Endzellen 30 mit verlängerten Stegen 31 vorgesehen sind. Bei dem Ausführungsbeispiel gemäß Fig. 1 ist jeweils jede zweite Endzelle 30 mit verlängerten Stegen 31 ausgestattet. Die Endzellen 30 mit verlängerten Stegen 31 stehen daher über einen axialen Rand der Gitterstruktur 10 vor.

Die verlängerten Stege 31 sind vorzugsweise durch ein Markerelement 32 miteinander gekoppelt. Das Markerelement 32 kann einstückig mit den verlängerten Stegen 31 ausgebildet sein. Insbesondere umfasst das Markerelement vorzugweise eine Öse 33 zur Aufnahme eines röntgensichtbaren Materials, wobei die Öse 33 einteilig mit der Gitterstruktur 10 ausgeformt sein kann.

Die verlängerten Stege 31 der Endzellen 30 münden ferner in erste Stegverbinder 21. Insgesamt weisen die Endzellen 30 jeweils erste Stegverbinder 21 auf. In Umfangsrichtung der Gitterstruktur 10 sind mehrere erste Stegverbinder 21 zueinander benachbart angeordnet und bilden einen ersten Verbinderring 23. Der erste Verbinderring 23 verläuft somit entlang des Endzellenrings.

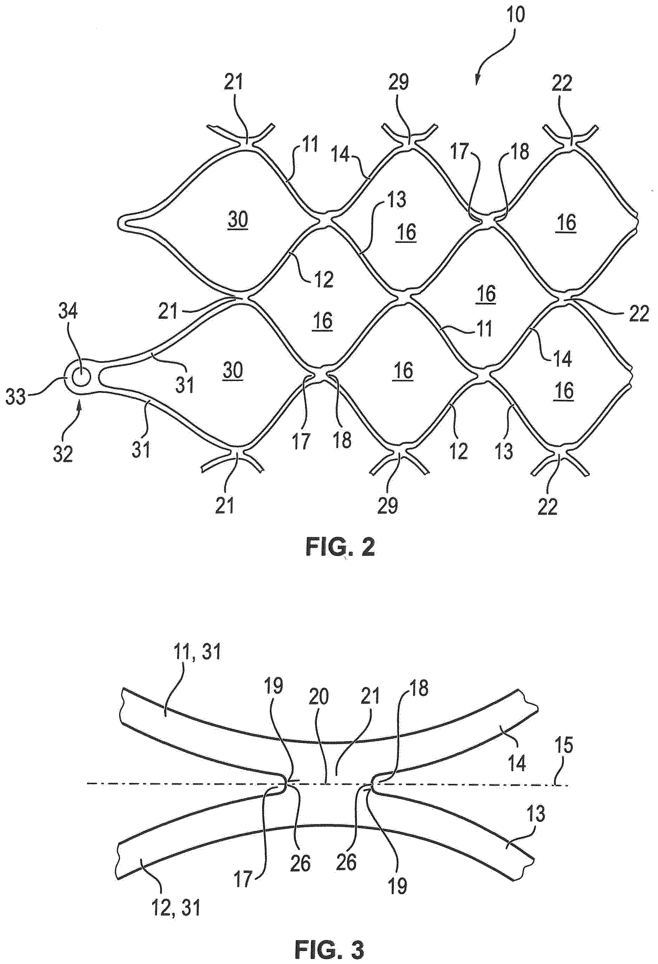

In Fig. 3 ist ein erster Stegverbinder 21 der Gitterstruktur 10 gemäß Fig. 1 im Detail gezeigt. Der erste Stegverbinder 21 verbindet zwei in Längsrichtung benachbart angeordnete Zellen 16, deren am ersten Stegverbinder 21 angeordnete Zellenspitzen 17, 18 jeweils fluchtend zueinander ausgerichtet sind. Mit anderen Worten besteht zwischen der ersten Zellenspitze 17 und der zweiten Zellenspitze 18 kein Versatz. Entsprechend ist die Verbinderachse 20 des ersten Stegverbinders 21 parallel zur Längsachse 15 der Gitterstruktur 10 ausgerichtet, wobei die Längsachse 15 in Fig. 3 durch eine strichpunktierte Linie eingezeichnet ist. Der erste Stegverbinder 21 behält neben dieser parallelen Ausrichtung zur Längsachse 15 seine Orientierung im Wesentlichen bei, auch wenn die Gitterstruktur 10 komprimiert und/oder expandiert wird. Bei der Komprimierung oder Expansion der Gitterstruktur 10 bewegen sich zwar die Stege 11, 12, 13, 14 relativ zueinander. Die Verbinderachse 20 bleibt jedoch parallel zur Längsachse 15 der Gitterstruktur 10 ausgerichtet. So kann der erste Stegverbinder 21 unabhängig vom Expansionsgrad der Gitterstruktur 10 gut längsaxiale Kräfte übertragen.

Zusätzlich zu den ersten Stegverbindern 21 weist die Gitterstruktur 10 zweite Stegverbinder 22 auf. Die zweiten Stegverbinder 22 umfassen eine Verbinderachse 20, die zumindest im Ruhezustand der Gitterstruktur 10 in einem Winkel zur Längsachse 15 der Gitterstruktur 10 ausgerichtet sind. Bei den zweiten Stegverbindern 22 weisen die am Stegverbinder 22 angeordneten Zellenspitzen 17, 18 jeweils einen Versatz in Umfangsrichtung zueinander auf. Dies ist in Fig. 2 erkennbar. Dadurch wird erreicht, dass im Unterschied zu der dauerhaft parallelen Ausrichtung der Verbinderachse 20 des ersten Stegverbinders 21, die Verbinderachse 20 des zweiten Stegverbinders 22 sich bei der Komprimierung und/oder Exansion der Gitterstruktur 10 mit unterschiedlichem Winkel zur Längsachse 15 der Gitterstruktur 10 ausrichtet. Bei der Komprimierung bzw. Expansion der Gitterstruktur 10 rotiert also der zweite Stegverbinder 22 um eine eigene Achse. Dies ermöglicht eine besonders hohe Flexibilität in Bereichen der Gitterstruktur 10, die mit zweiten Stegverbindern 22 ausgestattet sind.

Die ersten Stegverbinder 21 und die zweiten Stegverbinder 22 bilden einen ersten Verbinderring 21 bzw. einen zweiten Verbinderring 22. Vorzugsweise ist im Bereich der Endzellen 30 bzw. der Endzellenringe jeweils ein erster Verbinderring 23 angeordnet. Grundsätzlich ist die Gitterstruktur 10 gemäß Fig. 1 in mehrere Abschnitte unterteilt. Insbesondere weist die Gitterstruktur 10 zwei Endabschnitte 27 auf. Zwischen den Endabschnitten 27 erstreckt sich ein Mittelabschnitt 28. Vorzugsweise sind in den Endabschnitten 27 jeweils ausschließlich erste Stegverbinder 21 angeordnet. Der Mittelabschnitt 28 weist hingegen zweite Stegverbinder 22 auf. Konkret können mehrere zweite Verbinderringe 24 in längsaxialer Richtung der Gitterstruktur 10 unmittelbar benachbart zueinander angeordnet sein und den Mittelabschnitt 28 bilden. Die Endabschnitte 27 sind vorzugsweise jeweils durch einen einzigen ersten Verbinderring 23 gebildet.

Zwischen dem ersten Verbinderring 23 und einem zweiten Verbinderring 24 kann im Bereich des Übergangs zwischen dem Endabschnitt 27 und dem Mittelabschnitt 28 ein Übergangsverbinderring 25 vorgesehen sein. Der Übergangsverbinderring 25 ist aus Stegverbindern 29 gebildet, die ebenfalls rotationsfähig sind. Die Stegverbinder 29 des Übergangsverbinderrings 25 weisen also ähnliche Eigenschaften wie die zweiten Stegverbinder 22 auf. Allerdings ist die Rotationsfähigkeit der Stegverbinder 29 des Übergangsverbinderrings 25 geringer als die Rotationsfähigkeit der zweiten Stegverbinder 22. Die Winkel zwischen den Verbinderachsen 20 im Übergangsverbinderring 25 und der Längsachse 15 der Gitterstruktur 10 sind daher kleiner als die Winkel der Verbinderachsen 20 der zweiten Stegverbinder 22 und der Längsachse 15 der Gitterstruktur 10.

Bei dem Ausführungsbeispiel gemäß Fig. 1 folgt also ausgehend vom axialen Ende der Gitterstruktur 10 zunächst ein einziger erster Verbinderring 23. Der erste Verbinderring 23 weist erste Stegverbinder 21 auf, die bei der Expansion der Gitterstruktur 10 nicht rotieren. Unmittelbar auf den ersten Verbinderring 23 folgt ein Übergangsverbinderring 25. Der Übergangsverbinderring 25 umfasst Stegverbinder 29, die bei der Expansion der Gitterstruktur 10 leicht rotieren. Unmittelbar auf den Übergangsverbinderring 25 folgen zur Mitte der Gitterstruktur 10 hin mehrere zweite Verbinderringe 24. Die zweiten Verbinderringe 24 umfassen zweite Stegverbinder 22, die bei der Expansion der Gitterstruktur 10 stark rotieren. Konkret kann sich bei den zweiten Stegverbindern 22 eine Rotation in einem Winkelbereich von 0 bis 30 Grad, insbesondere 0 bis 25 Grad, insbesondere 0 bis 20 Grad, insbesondere 0 bis 15 Grad, einstellen.

Die Abfolge der einzelnen Verbinderringe ist gut in der Detailansicht gemäß Fig. 2 erkennbar. Insbesondere zeigt Fig. 2 deutlich, dass die Verbinderachse 20 der ersten Stegverbinder 21 im Bereich der Endzellen 30 parallel zur Längsachse 15 der Gitterstruktur 10 ausgerichtet ist. Dies ist gut dadurch zu sehen, dass die Zellenspitzen 17, 18 an den ersten Stegverbindern 21 fluchtend zueinander ausgerichtet sind. Bei zur Mitte der Gitterstruktur 10 hin folgenden Stegverbindern 22, 29 ist hingegen ein Versatz der Zellenspitzen 17, 18 erkennbar. Diese Stegverbinder, die zum Teil Stegverbinder 29 des Übergangsverbinderrings 25 und zum Teil zweite Stegverbinder 22 sind, rotieren bei der Komprimierung bzw. Expansion der Gitterstruktur 10.

Im Allgemeinen ändern die Stegverbinder 21, 22, 25 der Gitterstruktur 10 ihre Ausrichtung relativ zueinander sowohl in Umfangsrichtung, als auch in Längsrichtung bei der Expansion der Gitterstruktur 10 nicht. Vielmehr ändert sich nur bei den zweiten Stegverbindern 22 und ggf. den Stegverbindern 29 des Übergangverbinderrings 29 die Ausrichtung der Verbinderachse 20. Mit anderen Worten drehen die zweiten Stegverbinder 22 bei der Expansion der Gitterstruktur 10, behalten Ihre Relativposition mit Bezug auf die in Längsrichtung und Umfangsrichtung benachbarten ersten und/oder zweiten Stegverbindern 21, 22, 25 jedoch im Wesentlichen bei. Lediglich der Abstand zwischen den Stegverbindern 21, 22 ändert sich, wobei sich die Stegverbinder 21, 22 auf geradlinigen Bewegungslinien voneinander entfernen oder, bei Komprimierung der Gitterstruktur 10, einander annähern.

Fig. 2 zeigt außerdem deutlich das Markerelement 32, das bei dem hier gezeigten Ausführungsbeispiel an jeder zweiten Endzelle 30 vorgesehen ist. Das Markerelement 32 ist als Öse 33 ausgebildet, wobei in das Auge 34 der Öse 33 ein röntgensichtbares Material eingebettet werden kann. Derartige Materialien sind beispielsweise Gold oder Platin. Dies verbessert die Sichtbarkeit der Gitterstruktur 10 im implantierten Zustand. Indem nur jede zweite Endzelle 30 mit einem Markerelement 32 ausgestattet ist, wird die Komprimierbarkeit der Gitterstruktur 10 nicht beeinträchtigt.

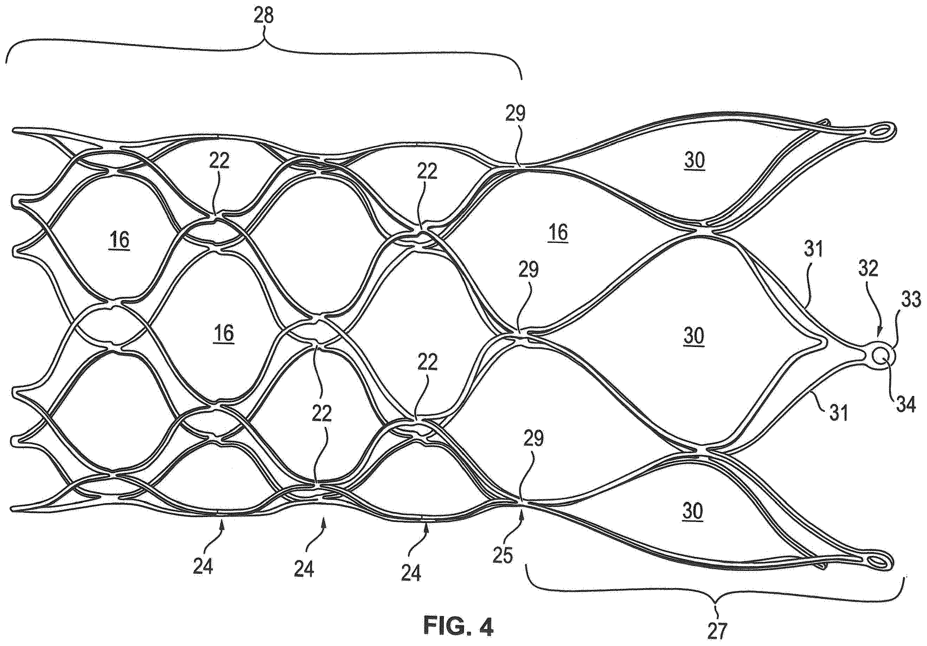

In Fig. 4 ist eine Seitenansicht der Gitterstruktur gemäß Fig. 1 gezeigt. Fig. 4 zeigt dabei den Ruhezustand, d.h. den vollständig expandierten Zustand der Gitterstruktur 10. Die Gitterstruktur 10 weist im Wesentlichen eine rohrförmige Gestalt auf. Zusätzlich ist in Fig. 4 erkennbar, dass die Gitterstruktur 10 im axialen Endabschnitt 27 radial nach außen aufgeweitet ist. Der Endabschnitt 27 weist also im Wesentlichen eine konische Form auf. Dies wird auch als "flairing" bezeichnet.

In Fig. 5 ist die Außenkontur der Gitterstruktur gemäß Fig. 1 im Ruhezustand schematisch dargestellt. Die Gitterstruktur 10 ist in drei Bereiche unterteilt, wobei zwei Endabschnitte 27 durch einen Mittelabschnitt 28 miteinander verbunden sind. Der Mittelabschnitt 28 weist eine zylinderförmige Außenkontur mit konstantem Querschnittsdurchmesser auf. Die Endabschnitte 27 weisen hingegen eine konusförmige Außenkontur auf, wobei sich die Endabschnitte 27 zu axialen Enden der Gitterstruktur hin aufweiten. Der Querschnittsdurchmesser der Gitterstruktur 10 erhöht sich also in den axialen Endabschnitten 27 in Richtung zum axialen Ende der Gitterstruktur 10. Vorzugsweise weist die Gitterstruktur 10 an den axialen Enden einen Querschnittsdurchmesser OD2 auf, der dem 1,3-fachen des Querschnittsdurchmessers OD1 im Mittelabschnitt 28 entspricht.

Die Länge der Endabschnitte 27 beträgt vorzugsweise zwischen 4 mm und 6 mm, insbesondere zwischen 4,5 mm und 5,5 mm, vorzugsweise 5 mm. Die Gesamtlänge der Gitterstruktur 10 bzw. des Stents beträgt vorzugsweise höchstens 35 mm, insbesondere höchstens 25 mm, insbesondere höchstens 15 mm.

Um die Flexibilität der Gitterstruktur 10, insbesondere des Mittelabschnitts 28, weiter zu erhöhen, können wenigstens die Zellen 16 im Mittelabschnitt 28 Stege 11, 12, 13, 14 aufweisen, die unterschiedliche Stegbreiten umfassen. Wie in Fig. 6 gezeigt ist, ist insbesondere vorgesehen, dass jeweils diametral gegenüberliegende Stege 11, 12, 13, 14 der Zelle 16 dieselbe Stegbreite aufweisen. So weisen der erste Steg 11 und der dritte Steg 13 eine erste Stegbreite S1 und der zweite Steg 12 und der vierte Steg 14 eine zweite Stegbreite S2 auf. Die erste Stegbreite S1 kann zwischen 30 µm und 50 µm, insbesondere zwischen 35 µm und 45 µm, vorzugsweise 40 µm betragen. Die zweite Stegbreite S2 kann vorzugsweise zwischen 25 µm und 40 µm, insbesondere zwischen 30 µm und 35 µm, vorzugsweise 32 µm, betragen.

Fig. 6 zeigt ferner einen Stegwinkel W zwischen dem ersten Steg 11 und der Längsachse 15 der Gitterstruktur 10. Der Stegwinkel W beträgt im Ruhezustand der Gitterstruktur vorzugsweise zwischen 40 Grad und 50 Grad, insbesondere zwischen 42 Grad und 48 Grad, vorzugsweise 44 Grad.

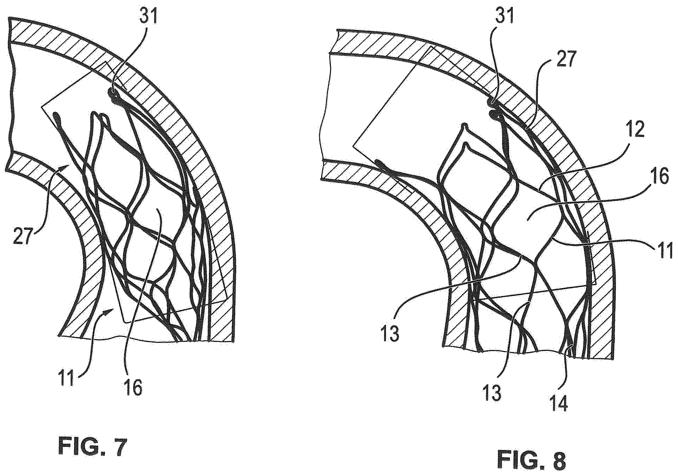

In den Fig. 7 und 8 ist ebenfalls eine Seitenansicht einer Gitterstruktur 10 gezeigt, wobei die Gitterstruktur 10 gemäß Fig. 7 einen zylinderförmigen Endabschnitt und die Gitterstruktur 10 gemäß Fig. 8 einen konisch aufgeweiteten Endabschnitt 27 aufweist. Die Fig. 7 und 8 zeigen die Gitterstruktur 10 jeweils im implantierten Zustand innerhalb eines gekrümmten Blutgefäßes. Im direkten Vergleich ist gut erkennbar, dass durch die konische Aufweitung der Endabschnitte 27 die Anpassung der Gitterstruktur 10 an den gewundenen Gefäßabschnitt verbessert wird. Konkret weitet sich der Endabschnitt 27 mit einer konischen Form radial aus, so dass die Stege 11, 12, 13, 14, im Endabschnitt 27 an der Gefäßwand anliegen. Bei dem zylinderförmigen Endabschnitt 27 erstrecken sich die Stege 11, 12, 13, 14 hingegen in das Gefäßlumen hinein und können so den Blutfluss behindern. Im Allgemeinen weist die Gitterstruktur 10 gemäß Fig. 8, die einen Endabschnitt 27 mit einer konischen Außenkontur aufweist, im Bereich des Endabschnitts 27 eine erhöhte Radialkraft auf. Dies verbessert die Anpassbarkeit und Fixierbarkeit der Gitterstruktur 10 im implantierten Zustand.

### Bezugszeichenliste

- 10: Gitterstruktur
- 11: Erster Steg
- 12: Zweiter Steg
- 13: Dritter Steg
- 14: Vierter Steg
- 15: Längsachse
- 16: Zelle
- 17: Erste Zellenspitze
- 18: Zweite Zellenspitze
- 19: Krümmung
- 20: Verbinderachse
- 21: Erster Stegverbinder
- 22: Zweiter Stegverbinder
- 23: Erster Verbinderring
- 24: Zweiter Verbinderring
- 25: Übergangsverbinderring
- 26: Scheitelpunkt
- 27: Endabschnitt
- 28: Mittelabschnitt
- 29: Stegverbinder des Übergangsverbinderrings 25
- 30: Endzelle
- 31: Verlängerter Steg
- 32: Markerelement
- 33: Öse
- 34: Auge

## Patentansprüche

1. Stent mit einer komprimierbaren und selbstexpandierbaren, rohrförmigen Gitterstruktur (10) aus Stegen (11, 12, 13, 14), die durch erste und zweite Stegverbinder (21, 22) einstückig miteinander verbunden sind und geschlossene Zellen (16) der Gitterstruktur (10) begrenzen, wobei jeweils vier Stege (11, 12, 13, 14) eine geschlossene Zelle (16) begrenzen und die Stegverbinder (21, 22) jeweils eine Verbinderachse (20) aufweisen, die dem kürzesten Abstand zwischen zwei Zellenspitzen (17, 18) von in Längsrichtung am Stegverbinder (21, 22) gegenüberliegenden Zellen (16) entspricht,
**dadurch gekennzeichnet, dass**
in einem Ruhezustand der Gitterstruktur (10) die Verbinderachsen (20) der ersten Stegverbinder (21) parallel und die Verbinderachsen (20) der zweiten Stegverbinder (22) in einem Winkel zu einer Längsachse (15) der Gitterstruktur (10) angeordnet sind.

2. Stent nach Anspruch 1,
**dadurch gekennzeichnet, dass**
jeweils in Umfangsrichtung der Gitterstruktur (10) benachbarte erste Stegverbinder (21) einen ersten Verbinderring (23) und jeweils in Umfangsrichtung der Gitterstruktur (10) benachbarte zweite Stegverbinder (22) einen zweiten Verbinderring (24) bilden.

3. Stent nach Anspruch 1 oder 2,
**dadurch gekennzeichnet, dass**
die ersten Stegverbinder (21), insbesondere der erste Verbinderring (23), an wenigstens einem axialen Ende der Gitterstruktur (10) angeordnet sind.

4. Stent nach einem der vorhergehenden Ansprüche,
**dadurch gekennzeichnet, dass**
an den axialen Enden der Gitterstruktur (10) jeweils ein, insbesondere ein einziger, erster Verbinderring (23) angeordnet ist.

5. Stent nach einem der vorhergehenden Ansprüche,
**dadurch gekennzeichnet, dass**
in Längsrichtung benachbarte Zellen (16) der Gitterstruktur (10) jeweils eine Zellenspitze (17, 18) mit einer Krümmung (19) umfassen, wobei die Krümmung (19) einen Scheitelpunkt (26) aufweist, der einen Endpunkt der Verbinderachse (20) bildet.

6. Stent nach einem der vorhergehenden Ansprüche,
**dadurch gekennzeichnet, dass**
die zweiten Stegverbinder (22) beim Übergang der Gitterstruktur (10) vom Ruhezustand in einen komprimierten Zustand rotieren derart, dass sich ein Winkel zwischen der Verbinderachse (20) und der Längsachse (15) der Gitterstruktur (10) ändert, insbesondere erhöht.

7. Stent nach einem der vorhergehenden Ansprüche,
**dadurch gekennzeichnet, dass**
in einem Mittelabschnitt (28) der Gitterstruktur (10), insbesondere zwischen den axialen Enden bzw. zwischen den ersten Verbinderringen (23), jeweils ein oder mehrere zweite Stegverbinder (22), insbesondere ein oder mehrere zweite Verbinderringe (24), angeordnet sind.

8. Stent nach einem der vorhergehenden Ansprüche,
**dadurch gekennzeichnet, dass**
zwischen einem ersten Verbinderring (23) und einem zweiten Verbinderring (24) ein Übergangsverbinderring (25) angeordnet ist, wobei die Stegverbinder (29) des Übergangsverbinderrings (25) in einem Ruhezustand der Gitterstruktur (10) einen kleineren Winkel mit der Längsachse der Gitterstruktur (10) bilden als die zweiten Stegverbinder (22) des zweiten Verbinderrings (24).

9. Stent nach einem der vorhergehenden Ansprüche,
**dadurch gekennzeichnet, dass**
die axialen Enden der Gitterstruktur (10) Endzellen (30) aufweisen, die jeweils zwei verlängerte Stege (31) umfassen, die durch ein Markerelement (32) miteinander verbunden sind.

10. Stent nach einem der vorhergehenden Ansprüche,
**dadurch gekennzeichnet, dass**
axiale Endabschnitte (27) der Gitterstruktur (10) konusförmig aufgeweitet sind.

11. Stent nach einem der vorhergehenden Ansprüche,
**dadurch gekennzeichnet, dass**
die axialen Endabschnitte (27) jeweils eine Länge aufweisen, die höchstens dem 0,3-fachen, insbesondere höchstens dem 0,25-fachen, insbesondere höchstens dem 0,15-fachen, der Gesamtlänge der Gitterstruktur (10) entspricht.

12. Stent nach einem der vorhergehenden Ansprüche,
**dadurch gekennzeichnet, dass**
die Stege (11, 12, 13, 14) der einzelnen Zellen (16) unterschiedliche Stegbreiten aufweisen.

13. Stent nach einem der vorhergehenden Ansprüche,
**dadurch gekennzeichnet, dass**
die Gitterstruktur (10) ein superelastisches Material, insbesondere eine Nickel-Titan-Legierung, vorzugsweise Nitinol, aufweist oder daraus besteht.

## Claims

1. Stent, with a compressible and self-expanding, tubular lattice structure (10) of filamentous elements (11, 12, 13, 14) which are connected integrally with each other via first and second filament connectors (21, 22) and delimit closed cells (16) of the lattice structure (10), wherein each closed cell (16) is delimited by four filamentous elements (11, 12, 13, 14), and the filament connectors (21, 22) each have a connector axis (20) that corresponds to the shortest distance between two cell tips (17, 18) of cells (16) that are longitudinally opposite one another on the filament connector (21, 22),
**characterised in that**,
when the lattice structure (10) is in a resting state, the connector axes (20) of the first filament connectors (21) are arranged in parallel and the connector axes (20) of the second filament connectors (22) are arranged at an angle to a longitudinal axis (15) of the lattice structure (10).

2. Stent according to claim 1,
**characterised in that**
respectively adjacent first filament connectors (21) in the circumferential direction of the lattice structure (10) form a first connector ring (23), and respectively adjacent second filament connectors (22) in the circumferential direction of the lattice structure (10) form a second connector ring (24).

3. Stent according to claim 1 or 2,
**characterised in that**
the first filament connectors (21), in particular the first connector ring (23), are arranged on at least at one axial end of the lattice structure (10).

4. Stent according to any one of the preceding claims,
**characterised in that**
a, in particular a single, first connector ring (23) is arranged on each axial end of the lattice structure (10).

5. Stent according to any one of the preceding claims,
**characterised in that**
longitudinally adjacent cells (16) of the lattice structure (10) each have a cell tip (17, 18) with a curvature (19), wherein the curvature (19) has an apex (26) that forms a terminal point of the connector axis (20).

6. Stent according to any one of the preceding claims,
**characterised in that**,
when the lattice structure (10) transitions from the resting state into a compressed state, the second filament connectors (22) rotate in such manner that an angle between the connector axis (20) and the longitudinal axis (15) of the lattice structure (10) changes, particularly increases.

7. Stent according to any one of the preceding claims,
**characterised in that**
one or more second bridge connectors (22), particularly one or more second connector rings (24), are arranged in a central portion (28) of the lattice structure (10), particularly between the axial ends and between the first connector rings (23), respectively.

8. Stent according to any one of the preceding claims,
**characterised in that**
a transition connector ring (25) is arranged between a first connector ring (23) and a second connector ring (24), wherein, when the lattice structure (10) is in a resting state, the filament connectors (29) of the transition connector ring (25) form a smaller angle with the longitudinal axis of the lattice structure (10) than the second filament connectors (22) of the second connector ring (24).

9. Stent according to any one of the preceding claims,
**characterised in that**
the axial ends of the lattice structure (10) have end cells (30), each of which include two elongated filaments (31) that are connected to each other by a marker element (32).

10. Device according to any one of the preceding claims,
**characterised in that**
axial end sections (27) of the lattice structure (10) are flared in the manner of a cone.

11. Stent according to any one of the preceding claims,
**characterised in that**
the axial end sections (27) each have a length which corresponds at most to a factor of 0.3, particularly at most to a factor of 0.25, more particularly at most to a factor of 0.15 of the total length of the lattice structure (10).

12. Stent according to any one of the preceding claims,
**characterised in that**
the filaments (11, 12, 13, 14) of the individual cells (16) have different filament widths.

13. Stent according to any one of the preceding claims,
**characterised in that**
the lattice structure (10) includes a superelastic material, particularly a nickel-titanium alloy, preferably Nitinol or consists thereof.

## Revendications

1. Stent avec une structure de treillis compressible et auto-expansible, de forme tubulaire (10) composée de côtés (11, 12, 13, 14), qui sont reliés les uns aux autres en une seule pièce par des premiers et seconds raccords de côté (21, 22) et limitent les cellules fermées (16) de la structure de treillis (10), quatre côtés (11, 12, 13, 14) limitant respectivement une cellule fermée (16) et les raccords de côté (21, 22) comportant respectivement un axe de raccord (20), qui correspond à l'intervalle le plus court entre deux pointes de cellule (17, 18) des cellules (16) opposées dans le sens longitudinal sur le raccord de côté (21, 22), **caractérisée en ce qu'**à un état de repos de la structure de treillis (10), les axes de raccord (20) des premiers raccords de côté (21) sont disposés parallèlement et les axes de raccord (20) des seconds raccords de côté (22) à un angle par rapport à un axe longitudinal (15) de la structure de treillis (10).

2. Stent selon la revendication 1, **caractérisée en ce que** dans le sens périphérique de la structure de treillis (10), les premiers raccords de côté voisins (21) forment respectivement un premier anneau de raccord (23) et les seconds raccords de côté voisins (22) dans le sens périphérique de la structure de treillis (10), respectivement un deuxième anneau de raccord (24).

3. Stent selon la revendication 1 ou 2, **caractérisée en ce que** les premiers raccords de côté (21), notamment le premier anneau de raccord (23), sont disposés sur au moins une extrémité axiale de la structure de treillis (10).

4. Stent selon l'une quelconque des revendications précédentes, **caractérisée en ce qu'**un premier anneau de raccord (23), notamment un seul, est respectivement disposé aux extrémités axiales de la structure de treillis (10).

5. Stent selon l'une quelconque des revendications précédentes, **caractérisée en ce que** dans le sens longitudinal, les cellules voisines (16) de la structure de treillis (10) comprennent respectivement une pointe de cellule (17, 18) avec une courbure (19), la courbure (19) comportant un point de sommet (26), qui forme un point extrême de l'axe de raccord (20).

6. Stent selon l'une quelconque des revendications précédentes, **caractérisée en ce que** les seconds raccords de côté (22) tournent lors du passage de la structure de treillis (10) de l'état de repos à un état comprimé de telle manière qu'un angle varie, en particulier augmente, entre l'axe de raccord (20) et l'axe longitudinal (15) de la structure de treillis (10).

7. Stent selon l'une quelconque des revendications précédentes, **caractérisée en ce que** dans une section médiane (28) de la structure de treillis (10), un ou plusieurs seconds raccords de côté (22), en particulier un ou plusieurs seconds anneaux de raccord (24), sont respectivement disposés notamment entre les extrémités axiales ou entre les premiers anneaux de raccord (23).

8. Stent selon l'une quelconque des revendications précédentes **caractérisée en ce qu'**un anneau de raccord de passage (25) est disposé entre un premier anneau de raccord (23) et un second anneau de raccord (24), les raccords de côté (29) de l'anneau de raccord de passage (25) formant dans un état de repos de la structure de treillis (10) un angle plus faible avec l'axe longitudinal de la structure de treillis (10) que les seconds raccords de côté (22) du deuxième anneau de raccord (24).

9. Stent selon l'une quelconque des revendications précédentes, **caractérisée en ce que** les extrémités axiales de la structure de treillis (10) comportent des cellules finales (30), qui comprennent respectivement deux côtés prolongés (31), qui sont reliés entre eux par un élément de repère (32).

10. Stent selon l'une quelconque des revendications précédentes, **caractérisée en ce que** les sections finales axiales (27) de la structure de treillis (10) sont élargies de forme conique.

11. Stent selon l'une quelconque des revendications précédentes, **caractérisée en ce que** les sections finales axiales (27) comportent respectivement une longueur, qui correspond au maximum à 0, 3 fois, notamment au maximum à 0, 25 fois, notamment au maximum à 0, 15 fois la longueur totale de la structure de treillis (10).

12. Stent selon l'une quelconque des revendications précédentes, **caractérisée en ce que** les côtés (11, 12, 13, 14) de chaque cellule (16) comportent des largeurs de côté différentes.

13. Stent selon l'une quelconque des revendications précédentes, **caractérisée en ce que** la structure de treillis (10) comporte un matériau superélastique, notamment un alliage nickel-titane, de préférence du nitinol, ou est composé de ceux-ci.
